# EUROPÄISCHE PATENTANMELDUNG

(11) **EP 4 666 980 A1**
(43) Veröffentlichungstag der Anmeldung: **24.12.2025**
(21) Anmeldenummer: 25183700.1
(22) Anmeldetag: 18.06.2025
(51) Int. Cl.: A61B 34/37, A61B 34/00, B25J 9/10, B25J 13/02

(54) **ROBOTERVORRICHTUNG**

(30) Priorität: 21.06.2024 DE 102024117645
(71) Anmelder: Karl Storz SE & Co. KG, 78532 Tuttlingen (DE)
(72) Erfinder: Hoffmann, Martin, 13187 Berlin (DE); Borutta, Matthias, 76287 Rheinstetten (DE)

(57) **Zusammenfassung**

Die Erfindung betrifft eine Robotervorrichtung (10) umfassend:
- einen proximalen Bewegungsarm (12), der um wenigstens eine Schwenkachse (S1) verschwenkbar ist,
- einen distalen Bewegungsarm (14), der gelenkig mit dem proximalen Bewegungsarm (12) gekoppelt ist,
wobei der distale Bewegungsarm (14) um wenigstens eine Drehachse (D1) relativ zum proximalen Bewegungsarm (12) drehbar ist, wobei die Drehachse (D1) winklig zu der Schwenkachse (S1) verläuft.

## Beschreibung

Die vorliegende Erfindung betrifft eine Robotervorrichtung, insbesondere als Teil eines medizinischen Robotersystems.

Telerobotiksysteme spielen eine immer wichtigere Rolle in der Medizin und ermöglichen es Chirurgen, minimal invasive Operationen präzise durchzuführen. Diese Systeme umfassen im Wesentlichen zwei Hauptkomponenten, die Master-Einheit und die Slave-Einheit. Bei der Master-Einheit handelt es sich in der Regel um ein Steuergerät, das von einem Chirurgen bedient wird und Bewegungen sowie Eingaben des Chirurgen erfasst. Bei der Slave-Einheit handelt es sich meistens um zumindest einen robotergestützten Effektor, der die Bewegungen der Master-Einheit präzise nachahmt und den chirurgischen Eingriff vornimmt. Der Chirurg steuert die Slave-Einheit über die Master-Einheit, wobei das System haptisches Feedback bietet, um dem Chirurgen ein Gefühl für beispielsweise Gewebewiderstände und - strukturen während eines chirurgischen Eingriffs zu vermitteln.

Herkömmliche Master- und/oder Slave-Einheiten von Robotersystemen weisen aufgrund ihres Konstruktionsaufbaus eine hohe Trägheit auf und/oder sind in ihrem Arbeitsraum, also dem dreidimensionalen Raum, innerhalb dessen die Master- und/oder Slave-Einheiten bewegt werden können, eingeschränkt, was insbesondere die Präzision und/oder die Benutzerfreundlichkeit eines solchen Robotersystems maßgeblich beeinträchtigt.

Der Erfindung liegt insbesondere aber nicht beschränkt darauf die Aufgabe zugrunde, eine Robotervorrichtung für den Einsatz in einem medizinischen Robotersystem vorteilhaft weiterzuentwickeln, insbesondere hinsichtlich einer Reduzierung einer Trägheit der Robotervorrichtung bei gleichzeitiger Erhaltung einer hohen Bewegungsfreiheit. Ferner ist es insbesondere eine Aufgabe der vorliegenden Erfindung, eine präzise und benutzerfreundliche Robotervorrichtung mit optimierten Eigenschaften bereitzustellen, sodass insbesondere das Verhältnis aus Größe der Robotervorrichtung und dem Arbeitsraum verbessert werden kann.

Diese Aufgabe wird erfindungsgemäß durch die Merkmale der unabhängigen Ansprüche gelöst. Weiterbildungen der Erfindung sind den abhängigen Ansprüchen zu entnehmen.

Die Erfindung betrifft eine Robotervorrichtung umfassend:
- einen proximalen Bewegungsarm, der um wenigstens eine Schwenkachse verschwenkbar ist;
- einen distalen Bewegungsarm, der gelenkig mit dem proximalen Bewegungsarm gekoppelt ist,
wobei der distale Bewegungsarm um wenigstens eine Drehachse relativ zum proximalen Bewegungsarm drehbar ist, wobei die Drehachse winklig zu der Schwenkachse verläuft.

Durch eine derartige Ausgestaltung kann eine vorteilhaft weiterentwickelte Robotervorrichtung bereitgestellt werden. Insbesondere weist eine solche Robotervorrichtung eine geringe Trägheit, bei gleichzeitiger Erhaltung einer hohen Bewegungsfreiheit auf. Zudem kann eine Robotervorrichtung mit einem vorteilhaften Verhältnis der Größe der Robotervorrichtung und dessen Arbeitsraum bereitgestellt werden, wodurch insbesondere die Präzision und/oder die Benutzerfreundlichkeit der Robotervorrichtung erhöht werden können.

Unter "Robotervorrichtung" soll insbesondere ein Bestandteil eines Robotersystems, insbesondere eine Unterbaugruppe und/oder eine Konstruktions- und/oder Funktionskomponente eines Robotersystems verstanden werden. Die Robotervorrichtung kann beispielsweise dazu eingerichtet sein, während eines chirurgischen Eingriffs präzise Bewegungen zuverlässig aufzunehmen und/oder auszuführen. Eine Robotervorrichtung kann beispielsweise Teil einer Master- und/oder Slave-Einheit eines Teleoperationssystems sein.

Unter "eingerichtet" soll speziell programmiert, vorgesehen, ausgelegt, ausgebildet und/oder ausgestattet verstanden werden. Darunter, dass ein Objekt zu einer bestimmten Funktion eingerichtet ist, soll ferner verstanden werden, dass das Objekt diese bestimmte Funktion in zumindest einem Anwendungs- oder Betriebszustand erfüllt oder ausführt.

Unter einem Bewegungsarm soll hierin insbesondere eine vorzugsweise längliche und/oder starre mechanische Struktur verstanden werden, welche insbesondere Teil eines Roboterarms oder eines Eingabearms ist und dazu eingerichtet sein kann, präzise und/oder intuitiv bewegt zu werden. Ein Bewegungsarm kann über eine begrenzte Anzahl von Freiheitsgraden verfügen. Die Struktur des Bewegungsarms kann je nach Anforderungen und Anwendungen variieren. Die Struktur des Bewegungsarms kann einteilig, monolithisch und/oder mehrteilig sein. Die Form des Bewegungsarms kann derart ausgestaltet sein, dass eine optimale Bewegungsfreiheit ermöglicht ist. Um ausreichende mechanische Eigenschaften, insbesondere im Hinblick auf eine Belastbarkeit und/oder Stabilität des Bewegungsarms zu gewährleisten, kann dieser vorzugsweise aus einem festen und/oder steifen Material wie beispielsweise Metall, Hochleistungspolymeren und/oder Verbundwerkstoffen oder aus einer weiteren vorteilhaften Materialkombination hergestellt sein. Die mechanische Struktur des Bewegungsarms kann Materialaussparungen aufweisen, die zur Gewichtsreduktion und/oder zur Verbesserung dessen mechanischen Eigenschaften beitragen können.

Bei dem proximalen Bewegungsarm kann es sich um eine Struktur handeln, die näher an einer Basis der Robotervorrichtung angeordnet ist und/oder sich ausgehend von der Basis erstreckt. Die Basis kann im Rahmen dieser Erfindung als eine grundlegende Konstruktion verstanden werden, an und/oder auf welcher der proximale Bewegungsarm mittelbar und/oder unmittelbar montierbar und/oder montiert ist und/oder von der aus sich der proximale Bewegungsarm aus erstreckt. Räumlich betrachtet kann der proximale Bewegungsarm einen unteren und/oder hinteren Bewegungsarm der Robotervorrichtung darstellen. Ein proximaler und/oder unterer Bewegungsarm kann sich dabei insbesondere auf einen Bewegungsarm beziehen, der im Vergleich zum distalen Bewegungsarm näher an der Basis der Robotervorrichtung angeordnet ist.

Der distale Bewegungsarm kann als ein Bewegungsarm der Robotervorrichtung verstanden werden, der im Vergleich zum proximalen Bewegungsarm weiter von der Basis der Robotervorrichtung entfernt angeordnet ist.

Dass der proximalen Bewegungsarm um wenigstens eine Schwenkachse verschwenkbar ist, soll hierin bedeuten, dass der proximale Bewegungsarm in eine oder mehrere Richtungen bewegt werden kann, um verschiedene Positionen zu erreichen. Die wenigstens eine Schwenkachse kann insbesondere in horizontaler Richtung verlaufen. Bei der Schwenkachse kann es sich vor allem um eine Bezugsachse handeln, die sich auf einen einzelnen Freiheitsgrad bezieht, der eine Schwenkbewegung einer Komponente um eine jene Schwenkachse ermöglicht. Die Schwenkbewegung des proximalen Bewegungsarms kann in vordefinierten Winkelschritten erfolgen, ist bevorzugt jedoch nahezu stufenlos und besonders bevorzugt stufenlos. Die Verbindungsstelle, die es dem proximalen Bewegungsarm erlaubt, sich um die Schwenkachse zu schwenken, kann hierin auch als Knickgelenk bezeichnet werden. Der proximale Bewegungsarm kann um zumindest 45°, bevorzugt zumindest 90° und besonders bevorzugt zumindest 120° um die Schwenkachse verschwenkbar sein.

Dass der distale Bewegungsarm, gelenkig mit dem proximalen Bewegungsarm gekoppelt ist, soll im Rahmen dieser Erfindung so verstanden werden, dass der proximale und der distale Bewegungsarm derart miteinander gekoppelt sind, dass der proximale und der distale Bewegungsarm unabhängig voneinander und/oder relativ zueinander bewegbar sind, währen der proximale Bewegungsarm mittelbar oder unmittelbar mit dem distalen Bewegungsarm verbunden bleibt.

Dass der distale Bewegungsarm um wenigstens eine Drehachse relativ zum proximalen Bewegungsarm drehbar ist, soll hierin bedeuten, dass der distale Bewegungsarm in eine oder mehrere Richtungen bewegt werden kann, um verschiedene Positionen zu erreichen. Der distale Bewegungsarm kann um eine Bezugsachse drehbar sein, die von der wenigstens einen Schwenkachse verschieden ist. Die Drehachse kann insbesondere senkrecht zu dem distalen Bewegungsarm verlaufen. Insbesondere wäre denkbar, dass die Drehachse für beliebige Schwenkstellungen des proximalen Bewegungsarms ihre Winkelausrichtung zu einer Schwerkraftrichtung beibehält und bevorzugt parallel zur Schwerkraftrichtung ausgerichtet bleibt. Bei der Drehachse kann es sich vor allem um eine Bezugsachse handeln, die sich auf einen einzelnen Freiheitsgrad bezieht, der eine Rotations- und/oder Drehbewegung einer Komponente um eine jene Drehachse ermöglicht. Die Drehbewegung des distalen Bewegungsarms kann in vordefinierten Winkelschritten erfolgen, ist bevorzugt jedoch nahezu stufenlos und besonders bevorzugt stufenlos. Die Verbindungsstelle, die es dem distalen Bewegungsarm erlaubt, sich um die Drehachse zu drehen, kann hierin auch als Drehgelenk bezeichnet werden. Der distale Bewegungsarm kann um zumindest 45°, bevorzugt zumindest 180° und besonders bevorzugt zumindest 360° um die Drehachse drehbar sein.

Erfindungsgemäß verläuft die Drehachse winklig zu der Schwenkachse. In anderen Worten verlaufen die Drehachse und die Schwenkachse in einem Winkel zueinander, welcher größer ist als 0° und kleiner ist als 180°. Der Winkel zwischen der Drehachse und der Schwenkachse kann insbesondere 70°, bevorzugt 80° und besonders bevorzugt 90° betragen. Der Winkel kann im Rahmen von Montage- und/oder Fertigungstoleranzen und/oder einer Abweichung von maximal 5° von einem rechten Winkel abweichen.

Gemäß einer Weiterbildung der Erfindung kann die Robotervorrichtung einen Interaktor und eine Kopplungseinheit umfassen, wobei die Kopplungseinheit ein mit dem Interaktor verbundenes erstes Kopplungselement, ein mit dem distalen Bewegungsarm verbundenes zweites Kopplungselement und eine Zugmittelanordnung mit wenigstens einem Zugmittel aufweist, welches mit dem ersten Kopplungselement und dem zweiten Kopplungselement gekoppelt ist, sodass eine Bewegung von einem der Kopplungselemente auf das andere der Kopplungselemente übertragbar ist. Der Interaktor und/oder das erste Kopplungselement können vorzugsweise basisnah, also auf und/oder an der Basis der Robotervorrichtung angeordnet sein. Eine derartige Ausgestaltung gestattet es, eine Masse der Robotervorrichtung näher an der Basis zu konzentrieren und auf diese Weise die Trägheit der Robotervorrichtung maßgeblich zu verringern. Durch die Zugmittelanordnung gelingt es Kräfte und/oder Bewegungen zwischen weiter voneinander beabstandeten Komponenten effizient zu übertragen und/oder aufzunehmen, ohne konstruktiv aufwändige und kostenintensive Getriebeanordnungen vorsehen zu müssen. Eine derartige Ausgestaltung kann ferner dazu beitragen, die Ergonomie zu verbessern und das Risiko von Ermüdungserscheinungen und/oder daraus resultierende Fehler eines Benutzers zu verringern.

Unter einem Interaktor kann insbesondere eine Einheit verstanden werden, die dazu eingerichtet ist, Kräfte und/oder Bewegungen aufzunehmen, zu übertragen, zu messen und/oder zu erzeugen. Zusätzlich oder alternativ kann der Interaktor einen physischen und/oder virtuellen Anschlag bereitstellen. Der Interaktor kann in einem Aktiv- und/oder in einem Passivmodus betrieben werden und/oder betreibbar sein. Je nach Anwendungsbereich kann der Interaktor ausschließlich in einem der beiden Modi betrieben werden und/oder betreibbar sein. Beispielsweise kann eine Robotervorrichtung einer Master-Einheit stets in einem Passivmodus betrieben werden. Eine Robotervorrichtung einer Slave-Einheit kann stets in einem Aktivmodus betrieben werden.

Unter einem "Passivmodus" soll hierin ein Betriebszustand verstanden werden, in dem die Robotervorrichtung passiv, also nicht aktiv angetrieben wird, sondern die Robotervorrichtung von externen Kräften, insbesondere von einem behandelnden Arzt und/oder Chirurgen, bewegt wird. Für diesen Modus kann der Interaktor und/oder die Robotervorrichtung eine Sensorik umfassen, die dazu eingerichtet ist, Bewegungsdaten der Vorrichtung zu erfassen, während sie manuell, also von Hand, bewegt wird. Die Bewegungen und/oder Steuersignale können beispielsweise von der Sensorik der Robotervorrichtung interpretiert und entsprechend in Anweisungen umgewandelt werden, welche Motoren und/oder Effektoren steuern. Die Steuerung der Motoren und/oder es Sektoren kann vorzugsweise in Echtzeit zu der Eingabe von Bewegungen, also dem Bewegen der Robotervorrichtung durch einen Benutzer, erfolgen. Im Passivmodus können ein oder mehrere Interaktoren Bewegungen von Gelenkabschnitten und/oder Bewegungsarmen durch einen oder mehrere veränderliche physische und/oder virtuelle Anschläge beschränken. Die Interaktoren können dazu eingerichtet sein eine taktile Rückmeldung und/oder einen Widerstand für einen Benutzer zu erzeugen.

Die Sensorik kann unterschiedliche, dem Fachmann vorteilhaft erscheinende Messvorrichtungen und/oder Sensoren umfassen, die dazu eingerichtet sind Bewegungen und/oder Bewegungsänderungen aufzuzeichnen und zu interpretieren. Die Sensoren können vorzugsweise an den unterschiedlichen Gelenkabschnitten und/oder den Dreh- Schwenk- und/oder Rotationsachsen vorgesehen sein. Die Sensorik kann insbesondere Encoder, Gyroskopsensoren, Inertialsensoren und/oder Hallsensoren handeln, die dazu eingerichtet sind Positionen, Bewegungen, Geschwindigkeiten und/oder Winkeländerungen der Bewegungsarme und/oder Kopplungselemente, vorzugsweise in Echtzeit zu detektieren und/oder aufzuzeichnen. Auf diese Weise ermöglicht die Eingabevorrichtung dem Benutzer eine intuitive und/oder präzise Steuerung der Robotervorrichtung und/oder den Effektoren.

Unter einem "Aktivmodus" soll hierin ein Betriebs Zustand verstanden werden, in dem die Robotervorrichtung aktiv angetrieben und/oder bewegt wird, um eine bestimmte Funktion auszuführen. Für diesen Modus kann der Interaktor und/oder die Robotervorrichtung wenigstens eine Antriebsquelle, beispielsweise in Form eines Elektromotors sowie dazugehöriges Getriebe umfassen, um die Robotervorrichtung, und/oder Abschnitte derer aktiv anzutreiben und/oder zu bewegen. Der Interaktor und/oder Robotervorrichtung kann in dem aktiven Betriebsmodus insbesondere automatisch und oder autonom navigieren. Auch in dem aktiven Betriebsmodus kann die Sensorik verwendet werden, um Feedback über die Umgebung der Robotervorrichtung zu liefern, seine Bewegungen zu überwachen und/oder sicherzustellen, dass er seine Aufgaben ordnungsgemäß ausführt.

Je nach Bedarf und/oder den Anforderungen der jeweiligen Aufgabe und/oder Eingabe des Benutzers, kann die Robotervorrichtung zwischen dem aktiven Betriebsmodus und dem passiven Betriebsmodus umschalten und/oder umgeschaltet werden. In Teleoperationssystemen kann vorzugsweise die Master-Einheit in einem passiven Betriebsmodus betrieben werden. Die Slave-Einheit kann vorzugsweise in einem aktiven Betriebsmodus betrieben werden.

Eine Kopplungseinheit kann hierin als eine Vorrichtung und/oder ein System verstanden werden, die dazu eingerichtet ist, unterschiedliche Komponenten der Robotervorrichtung mechanisch und/oder bewegungsübertragbar miteinander zu koppeln, was es ermöglicht, dass die gekoppelten Komponenten im Betrieb zuverlässig und funktional zusammenarbeiten und/oder miteinander interagieren.

Unter einem Kopplungselement kann insbesondere eine Komponente verstanden werden, die dazu eingerichtet ist, mit einemweiteren Kopplungselement gekoppelt zu werden, um deren Interaktion und/oder Funktion zu ermöglichen. Hierin kann das Kopplungselement eine Art Trommel umfassen, die dazu eingerichtet ist, Bewegungen des Zugmittels zu steuern und/oder aufzunehmen, indem das Kopplungselement das Zugmittel aufwickelt und/oder abwickelt. Das Kopplungselement kann vorzugsweise einen runden Querschnitt aufweisen und/oder zylindrisch ausgestaltet sein, sodass das Zugmittel gleichmäßig und/oder sicher auf unterschiedlichen Bereichen der Trommel zumindest abschnittsweise aufwickelbar und/oder abwickelbar ist. Das Zugmittel kann beispielsweise ein Seil, einen Riemen und/oder eine Kette umfassen. Gemäß einiger Ausführungsformen kann das Zugmittel eine Mehrzahl an Seilen, Riemen und/oder Ketten umfassen.

Während das erste Kopplungselement basisnah angeordnet sein kann, kann das zweite Kopplungselement von der Basis und/oder dem ersten Kopplungselement entfernt angeordnet sein. Zwischen dem ersten Kopplungselement und dem zweiten Kopplungselement kann ein Abstand vorgesehen sein, der mindestens der Länge des proximalen Bewegungsarms entspricht.

Unter einer Zugmittelanordnung soll hierin eine Struktur und/oder ein Mechanismus, verstanden werden dazu eingerichtet ist, um Kräfte und/oder Bewegungen des distalen Bewegungsarms auf den Interaktor und/oder das erste Kopplungselement zu übertragen und/oder umgekehrt. Die Zugmittelanordnung kann mehrere Zugmittel umfassen. Das oder die Zugmittel kann/können derart mit den Kopplungselementen wechselwirken, dass ein Aufwickeln wenigstens eines Zugmittels zu einem Abwickeln desselben Zugmittels auf dem zweiten Kopplungselement führt und umgekehrt.

In einigen Ausführungsformen kann ein erster Endabschnitt des Zugmittels zumindest teilweise an und/oder um das erste Kopplungselement und ein zweiter Endabschnitt des Zugmittels zumindest teilweise an und/oder um das zweite Kopplungselement gewickelt sein, sodass das erste Kopplungselement und das zweite Kopplungselement interdependent drehbar gekoppelt sind. Auf diese Weise kann eine effiziente und/oder synchronisierte Koordination und/oder Zusammenarbeit der Kopplungselemente erreicht werden.

"Interdependent drehbar gekoppelt" soll insbesondere bedeuten, dass das erste Kopplungselement derart mit dem zweiten Kopplungselement gekoppelt sein kann, dass sich die beiden Kopplungselemente insbesondere hinsichtlich ihrer Position und/oder Ausrichtung gegenseitig beeinflussen. Dabei ist die Position und/oder das Verhalten eines jeden Kopplungselements von der Position und/oder dem Verhalten eines jeden anderen in der Kopplungseinheit gekoppelten Kopplungselements abhängig. Die Bewegungen der beiden Kopplungselemente können in die gleiche Richtung. In anderen Worten können sich das erste Kopplungselement und das interdependent mit dem ersten Kopplungselement gekoppelte zweite Kopplungselement synchron bewegen. Die Kopplungselemente können gleich oder unterschiedlich ausgebildet sein. Die Kopplungselemente können ein Übersetzungsverhältnis bereitstellen. Die Kopplungselemente können sich insbesondere in ihrer Größe und/oder ihres Durchmessers unterscheiden.

Um das Zugmittel bzw. die Zugmittel in der richtigen Position zu halten und dessen Bewegung zu führen, um eine effiziente Kraftübertragung zu gewährleisten und den Bauraum effizient und/oder platzsparend auszunutzen, kann die Zugmittelanordnung eine Mehrzahl von Führungsmitteln, vorzugsweise in Form von Führungsrollen, umfassen. Die Führungsmittel können wenigstens eine Nut und/oder Rille aufweisen, um das Zugmittel zu führen und/oder Bewegungen in einer gewünschten Richtung umzulenken. Zusätzlich oder alternativ können die Führungsmittel Seilscheiben und/oder Führungskanäle, beispielsweise in Form von Führungszylindern umfassen. Die Anordnung der Führungsrollen kann das Zugmittel entlang und/oder auf unterschiedlichen Bezugsebenen führen, die zueinander winklig sind. In anderen Worten können die Führungsmittel derart angeordnet sein, dass sie das bzw. die Zugmittel auf oder parallel zu einer ersten Bezugsebene, einer zweiten Bezugsebene und/oder wenigstens einer dritten Bezugsebene führen, wobei sich die Bezugsebenen schräg und/oder senkrecht zueinander erstrecken können. Durch den Einsatz mehrerer Führungsmittel können für eine Bewegung der Robotervorrichtung erforderliche Kräfte verringert werden. Dadurch kann unter anderem die Benutzerfreundlichkeit und/oder Effizienz der Robotervorrichtung erhöht werden.

Die Führungsmittel können derart angeordnet sein, dass sich das Zugmittel zumindest abschnittsweise überkreuzt. Das Zugmittel kann einzelne Komponenten, darunter beispielsweise Seile, Riemen, Ketten und/oder ähnliche Bauteile und/oder eine Kombination daraus umfassen, die sich jeweils mit sich selbst und/oder mit einer anderen Komponente des Zugmittels überkreuzen. Durch das Überkreuzen können ungleichmäßige Lastverteilungen entlang der Führungsstrecke ausgeglichen und das Zugmittel platzsparend geführt werden.

Das erste Kopplungselement und das zweite Kopplungselement können jeweils um Rotationsachsen drehbar sein, welche winklig zueinander angeordnet sind, und zwar insbesondere unter einem Winkel größer als 0° und kleiner als 180°, vorzugsweise von größer als 80° und kleiner als 100° und besonders bevorzugt von 90°. Zueinander winklige Rotationsachsen gestatten eine kompakte Ausgestaltung der Robotervorrichtung mit mehreren Freiheitsgraden und/oder eine effiziente Nutzung des Bauraums. Durch die Anordnung in unterschiedlichen Winkeln können mittels der Robotervorrichtung komplexe anwendungsspezifische Bewegungen präzise ausgeführt werden. In einigen Ausführungsformen können die Rotationsachsen koaxial zu der Schwenkachse und/oder der Drehachse verlaufen.

Gemäß einer Weiterbildung der Erfindung kann der distale Bewegungsarm relativ zum proximalen Bewegungsarm um eine weitere Schwenkachse schwenkbar sein. Die weitere Schwenkachse kann parallel zur Schwenkachse verlaufen. Die weitere Schwenkachse kann vorzugsweise in einem 90° Winkel also orthogonal zu der Drehachse verlaufen. Der Winkel kann im Rahmen von Montage- und/oder Fertigungstoleranzen und/oder einer Abweichung von maximal 5° von einem rechten Winkel abweichen.

Durch die weitere Schwenkachse kann die Anzahl der Freiheitsgrade der Robotervorrichtung erhöht werden, was dazu führt, dass die Robotervorrichtung in mehr Richtungen und mit größerer Flexibilität bewegt werden kann. **In** anderen Worten kann der Arbeitsbereich der Robotervorrichtung vergrößert werden. Auf diese Weise können komplexere und präzise Bewegungen mit hoher Genauigkeit ausgeführt werden, was insbesondere in der Chirurgie zu besseren Behandlungsergebnissen führen kann. Die Verbindungsstelle, die es dem proximalen Bewegungsarm erlaubt, sich um die weitere Schwenkachse zu schwenken, kann hierin auch als weiteres Knickgelenk bezeichnet werden.

In einigen Ausführungsformen kann die Robotervorrichtung einen Nebenarm umfassen, welcher zumindest abschnittsweise, vorzugsweise größtenteils, entlang und/oder parallel des proximalen Bewegungsarms verläuft und von diesem beabstandet ist. Bei dem Nebenarm kann es sich um ein oder mehrere Bewegungselemente handeln, die dazu eingerichtet sind, die Basis mit dem proximalen Bewegungsarm und/oder dem distalen Bewegungsarm zu koppeln. Der Nebenarm kann bei einer Schwenkbewegung des distalen Bewegungsarms um die weitere Schwenkachse relativ zum proximalen Bewegungsarm bewegbar sein. Der Nebenarm kann unter anderem die Stabilität der Robotervorrichtung verbessern und eine präzisere Bewegungskontrolle ermöglichen. Ferner können Lasten auf den proximalen Bewegungsarm und den Nebenarm verteilt werden, was sich positiv auf die Lebensdauer und/oder die Zuverlässigkeit der Robotermechanik auswirken kann. Ferner kann der Nebenarm dazu beitragen, die Stabilität der Robotervorrichtung zu erhöhen und unerwünschte Bewegungen zu minimieren.

Gemäß einiger Ausführungsformen kann die Zugmittelanordnung zumindest teilweise an und/oder in dem Nebenarm angeordnet sein. Auf diese Weise kann eine effiziente und/oder wenig störanfällige und/oder geschützte Anordnung der Zugmittelanordnung gewährleistet werden. Eine derartige Anordnung kann eine Wartung und/oder Fehlerbehebung der Zugmittelanordnung erleichtern.

Gemäß einer Weiterbildung der Erfindung kann die Robotervorrichtung eine Eingabevorrichtung umfassen, die dazu eingerichtet ist, eine Bewegung und/oder Befehle eines Benutzers aufzunehmen. Die Eingabevorrichtung kann an einem distalen Endabschnitt des distalen Bewegungsarms angeordnet sein. Bei der Eingabevorrichtung kann es sich vorzugsweise um ein Gerät und/oder eine Schnittstelle handeln, die dazu eingerichtet ist, Daten und/oder Befehle, auf die Robotervorrichtung zu übertragen. Die Eingabevorrichtung kann insbesondere einen Joystick und/oder Steuerhebel umfassen, der dazu eingerichtet ist, die Robotervorrichtung in unterschiedliche Richtungen und Positionen präzise zu steuern und/oder Steuersignale an ein Computersystem und/oder andere Geräte zu übermitteln. Die Eingabevorrichtung kann vorzugsweise Bestandteil einer Master-Einheit sein und/oder mit einer solchen funktionsfähig koppelbar sein.

In alternativen Ausführungsformen kann statt der Eingabevorrichtung ein Effektor an dem distalen Ende des distalen Bewegungsarms vorgesehen sein. Der Effektor kann insbesondere ein medizinisches Instrument umfassen. Bei dem medizinischen Instrument kann es sich um einen beliebigen, dem Fachmann als sinnvoll erscheinenden Effektor, insbesondere in Form eines medizinischen und/oder chirurgischen Instruments handeln, welches bevorzugt durch die Robotervorrichtung getragen und durch diese bewegbar sein kann. Das medizinische Instrument kann mit dem distalen Bewegungsarm fest oder lösbar verbunden sein oder Teil des distalen Bewegungsarms sein. Das medizinische Instrument kann beispielsweise eine laparoskopische Einheit, ein Endoskop, ein Mikroskop, ein Exoskop, ein Skalpell, einen Bohrer, einen Schaber, eine Klammer, eine Zange, eine Schere, eine Spritze, einen Katheter und/oder weitere, dem Fachmann als sinnvoll erscheinende Einheiten aufweisen, welche mittels der Robotervorrichtung antreibbar und/oder ansteuerbar sein können. Der Effekor kann vorzugsweise Bestandteil einer Slave-Einheit sein und/oder mit einer solchen funktionsfähig koppelbar sein.

In einigen Ausführungsformen kann die Robotervorrichtung einen Ausrichtungsmechanismus umfassen, der dazu eingerichtet ist, zumindest bei einer Drehbewegung des distalen Bewegungsarms um die Drehachse eine entsprechende Drehbewegung der Eingabevorrichtung und/oder des Effektors zu verhindern und/oder zu kompensieren. Dadurch kann eine gewünschte und/oder präzise Positionierung der Eingabevorrichtung und/oder des Effektors erleichtert und/oder zuverlässig umgesetzt werden, was Fehler reduziert und/oder die Handhabung vereinfacht.

Der Ausrichtungsmechanismus kann wenigstens zwei Umlenkrollen umfassen, wobei eine erste Umlenkrolle an einem proximalen Endabschnitt des distalen Bewegungsarms angeordnet ist, und wobei eine zweite Umlenkrolle an dem distalen Endabschnitt des distalen Bewegungsarms angeordnet ist. Ferner kann der Ausrichtungsmechanismus wenigstens ein Zugelement umfassen, das zwischen und/oder um die wenigstens zwei Umlenkrollen gespannt ist, um eine Ausrichtung der Umlenkrollen zu synchronisieren.

Bei einer Umlenkrolle kann es sich um eine Scheibe handeln, die an ihrer radialen Umfangsfläche wenigstens eine Nut und/oder eine Rille umfasst, um das Zugelement zu führen.

Gemäß einiger Ausführungsformen kann die Eingabevorrichtung und/oder der Effektor drehfest an der zweiten Umlenkrolle angeordnet sein. Dadurch kann sich der distale Bewegungsarm bewegen, ohne dass sich dabei auch die Eingabevorrichtung und/oder der Effektor mitbewegt. Dadurch wird insbesondere die Bewegungsfreiheit verbessert, was insgesamt zu einer effizienten und/oder benutzerfreundlichen Leistung der Robotervorrichtung beiträgt.

In einer Weiterbildung der Erfindung kann die erste Umlenkrolle relativ zur Drehachse stationär und die zweite Umlenkrolle relativ zu der Drehachse bewegbar sein. Eine derartige Ausgestaltung ermöglicht eine drehfeste Anordnung der Eingabevorrichtung und/oder des Effektors an der zweiten Umlenkrolle. Damit kann die Eingabevorrichtung und/oder der Effektor unabhängig vom Rest der Robotervorrichtung bewegt werde und/oder umgekehrt. In anderen Worten kann die Ausrichtung der Eingabevorrichtung und/oder des Effektors von einer Bewegung des distalen Bewegungsarms und/oder der Robotervorrichtung um eine der Schwenkachsen unbeeinflusst bleiben.

Das wenigstens eine Zugelement kann an jeweils zumindest einem Punkt an den Umlenkrollen fixiert sein. Diese Fixierung gewährleistet, dass die Ausrichtung der Eingabevorrichtung und/oder des Effektors nach und/oder während einer Bewegung des distalen Bewegungsarms und/oder der Robotervorrichtung um eine Drehachse unverändert bleibt. Der Benutzer und/oder ein Stellmotor müssen keine zusätzliche Kraft aufwenden, um die Ausrichtung der Eingabevorrichtung und/oder des Effektors während einer Benutzung beizubehalten.

Zusätzlich oder alternativ zu dem Ausrichtungsmechanismus kann die Robotervorrichtung einen weiteren Ausrichtungsmechanismus umfassen. Der weitere Ausrichtungsmechanismus kann insbesondere eine Gelenkvorrichtung umfassen, die dazu eingerichtet ist, die Eingabevorrichtung und/oder den Effektor unabhängig von dem Ausrichtungsmechanismus bzw. einer Bewegung des distalen Bewegungsarms um eine Ausrichtungsachse auszurichten. Dafür kann der weitere Ausrichtungsmechanismus eine Schub- und/oder Zugstange umfassen. Bei einer Schub- und/oder Zugstange kann es sich insbesondere um einen oder mehrere Stäbe handeln, die dazu eingerichtet sind, eine Druckkraft und/oder eine Zugkraft auf die Gelenksvorrichtung auszuüben. Eine derartige Ausgestaltung hat den Vorteil, dass eine gewünschte Ausrichtung und/oder Orientierung der Eingabevorrichtung und/oder des Effektors auch nach und/oder während einer Bewegung des distalen Bewegungsarms und/oder der Robotervorrichtung beibehalten werden kann.

Die Robotervorrichtung und/oder einzelne Komponenten derer können eine Verkleidung umfassen. Die Verkleidung kann insbesondere dazu eingerichtet sein, Komponenten, die zumindest abschnittsweise von der Verkleidung umgeben werden, gegen äußere Einflüsse wie Staub, Feuchtigkeit und/oder mechanische Beschädigungen zu schützen. Die Verkleidung kann aus verschiedenen Materialien wie Kunststoff, Metall oder Gummi bestehen. Die Verkleidung kann so konfiguriert sein, dass es einfach mit wenigstens einem anderen Gehäuse oder System verliersicher verbunden werden kann.

Zusätzlich oder alternativ zu dem Interaktor, kann die Robotervorrichtung wenigstens ein physisches und/oder virtuelles Anschlagelement umfassen, das dazu eingerichtet ist, Bewegungen der einzelnen Komponenten um eine jeweilige Schwenk- und/oder Drehachse der jeweiligen Komponenten zuzulassen und/oder ab einem bestimmten Weg oder Betrag zu begrenzen. Unter einem virtuellen Anschlagelement kann beispielsweise eine Programmierung der Robotervorrichtung verstanden werden. Auf diese Weise können einfach und unkompliziert Bewegungsbereiche definiert und/oder geändert werden, ohne dass zusätzliche Hardware installiert werden muss. Auf diese Weise können unerwünschte Belastungen bzw. übermäßige Bewegungen und damit potenzielle Schäden der Robotervorrichtung verhindert werden. Das wenigstens eine Anschlagelement kann als eine Art Schutzmechanismus betrachtet werden, um die Integrität und Zuverlässigkeit der Robotervorrichtung zu gewährleisten.

Gemäß einer Weiterbildung kann die Robotervorrichtung die Basis umfassen. Die Basis kann um eine weitere Drehachse rotierbar sein. Die weitere Drehachse schafft einen weiteren Freiheitsgrad, wodurch das Arbeitsfeld der Robotervorrichtung zusätzlich vergrößert wird.

Die Erfindung betrifft ferner ein medizinisches Robotersystem, welches wenigstens eine Robotervorrichtung der vorangehend beschriebenen Art umfasst.

Durch eine derartige Ausgestaltung kann eine vorteilhaft weiterentwickeltes Robotersystem bereitgestellt werden. Insbesondere weist eine solches Robotersystem eine geringe Trägheit, bei gleichzeitiger Erhaltung einer hohen Bewegungsfreiheit auf. Zudem kann ein Robotersystem mit einem vorteilhaften Verhältnis der Größe des Robotersystems und dessen Arbeitsraum bereitgestellt werden, wodurch insbesondere die Präzision und/oder die Benutzerfreundlichkeit des Robotersystems erhöht werden können.

Das Robotersystem kann zumindest einen Elektronikschrank, insbesondere ein Elektronik-Rack, beispielsweise zur Aufnahme zusätzlicher Geräte wie eines HF-Generators, eine Anzeigeeinheit, eine Patientenliege, eine Aufbewahrungseinheit für verschiedene medizinische Instrumente und/oder weitere, dem Fachmann als sinnvoll erscheinende Einheiten aufweisen. Das medizinische Robotersystem kann mehrere Robotervorrichtungen aufweisen, insbesondere eines für jeden Arm eines Bedieners. In einem Betriebszustand kann ein medizinisches Instrument und/oder eine Bedienkonsole von jeweils einer Robotervorrichtung getragen und durch diese bewegbar sein.

Das Robotersystem kann außerdem eine Anzeigeeinheit umfassen, die dazu eingerichtet ist, ein Bild, insbesondere ein Bewegtbild eines zu bearbeitenden Objektbereichs, für einen Benutzer anzuzeigen. Bei dem Objektbereich kann es sich insbesondere um einen Bereich, der physiologische Bestandteile umfasst, wie beispielsweise Gewebe, Blut oder dergleichen, handeln. Der Objektbereich liegt beispielsweise innerhalb einer natürlichen oder künstlich geschaffenen Kavität. Solche Kavitäten sind in etwa der Bauchraum, der Darm, die Blase, die Niere oder dergleichen. Allerdings könnte auch offenes Gewebe als Objektbereich dienen. Die Anzeigeeinheit kann einen Bildschirm und/oder Steuerungselektronik umfassen. Die Anzeigeeinheit kann einen Computer und/oder Prozessor und/oder Speicher und/oder Arbeitsspeicher und/oder Anschlüsse und/oder eine Datenschnittschnittstelle zum Empfangen, Verarbeiten und Ausgeben von unverarbeiteten, vorverarbeiteten und/oder verarbeiteten Bilddaten und/oder Darstellungsdaten umfassen.

Die erfindungsgemäßen Vorrichtungen und Systeme sollen hierbei nicht auf die oben beschriebene Anwendung und Ausführungsform beschränkt sein. Insbesondere können diese zu einer Erfüllung einer hierin beschriebenen Funktionsweise eine von einer hierin genannten Anzahl von einzelnen Elementen, Bauteilen und Einheiten abweichende Anzahl aufweisen. Zudem sollen bei den in dieser Offenbarung angegebenen Wertebereichen auch innerhalb der genannten Grenzen liegende Werte als offenbart und als beliebig einsetzbar gelten.

Im Folgenden wird die vorliegende Erfindung anhand der beigefügten Figuren beispielhaft beschrieben. Die Zeichnungen, die Beschreibung und die Ansprüche enthalten zahlreiche Merkmale in Kombination. Der Fachmann wird die Merkmale zweckmäßigerweise auch einzeln betrachten und im Rahmen der Ansprüche sinnvoll in Kombination verwenden.

Falls von einem bestimmten Objekt mehr als ein Exemplar vorhanden ist, ist ggf. nur eines davon in den Figuren und in der Beschreibung mit einem Bezugszeichen versehen. Die Beschreibung dieses Exemplars kann entsprechend auf die anderen Exemplare von dem Objekt übertragen werden. Sind Objekte insbesondere mittels Zahlenwörtern, wie beispielsweise erstes, zweites, drittes Objekt etc. benannt, dienen diese der Benennung und/oder Zuordnung von Objekten. Demnach können beispielsweise ein erstes Objekt und ein drittes Objekt, jedoch kein zweites Objekt umfasst sein. Allerdings könnten anhand von Zahlenwörtern zusätzlich auch eine Anzahl und/oder eine Reihenfolge von Objekten ableitbar sein.

Es zeigen:
- Fig. 1: ein medizinisches Robotersystem mit einer Robotervorrichtung zum Steuern eines medizinischen Instruments,
- Fig. 2: eine perspektivische Darstellung einer Detailansicht der Robotervorrichtung,
- Fig. 3: eine perspektivische Darstellung der Robotervorrichtung ohne Verkleidung,
- Fig. 4: eine perspektivische Darstellung der Robotervorrichtung ohne Verkleidung aus einem anderen Blickwinkel,
- Fig. 5: eine perspektivische Darstellung der Robotervorrichtung mit einer Zugmittelanordnung,
- Fig. 6: eine schematische Darstellung der Zugmittelanordnung,
- Fig. 7: eine perspektivische Darstellung des distalen Bewegungsarms in einer ersten Position,
- Fig. 8: eine perspektivische Darstellung des distalen Bewegungsarms in einer zweiten Position,
- Fig. 9: eine schematische Darstellung distalen Bewegungsarms in der ersten Position,
- Fig. 10: eine schematische Darstellung des distalen Bewegungsarms in der zweiten Position,
- Fig. 11: eine Seitenansicht einer Robotervorrichtung gemäß einer weiteren Ausführungsform in einer ersten Position, und
- Fig. 12: eine Seitenansicht der Robotervorrichtung gemäß Fig. 11 in einer zweiten Position.

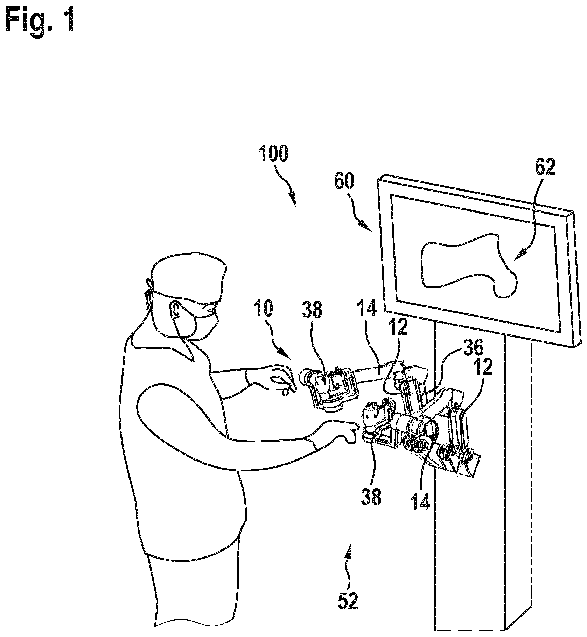

Fig. 1 zeigt ein medizinisches Robotersystem 100. Das Robotersystem 100 weist eine Master-Einheit 52 und zwei Robotervorrichtungen 10 auf, wobei Master-Einheit 52 dazu eingerichtet ist, eine hierin nicht gezeigte Slave-Einheit durch einen Benutzer anzusteuern. Die Robotervorrichtungen 10 der Slave-Einheit sind jeweils mit einer, an einem distalen Bewegungsarm angeordneten Eingabevorrichtungen 38 ausgestattet, die dazu eingerichtet sind, eine Bewegung und/oder einen Befehl des Benutzers aufzunehmen. An dieser Stelle sei darauf hingewiesen, dass die hier in gezeigten Eingabevorrichtungen 38 lediglich schematisch dargestellt sind. Es versteht sich von selbst, dass je nach Anwendungsbereich unterschiedliche Eingabevorrichtungen 38 eingesetzt werden können. Das medizinische Robotersystem 100 weist ferner eine Anzeigeeinheit 60 auf, die dazu eingerichtet ist, dem Benutzer ein Bild 62 eines zu bearbeitenden Objektbereichs anzuzeigen.

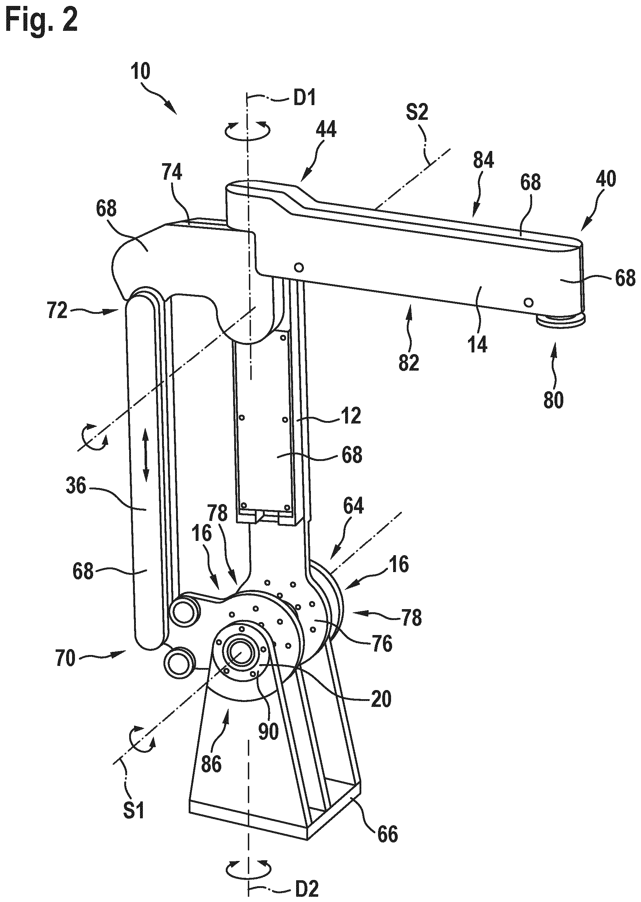

Die jeweiligen Robotervorrichtungen 10 weisen eine Vielzahl an Freiheitsgraden auf, wodurch ein besonders großer bzw. breiter Arbeitsbereich abgedeckt wird. Hinsichtlich der konstruktiven Ausgestaltung der Roboterverrichtungen 10 kann auf die nachfolgenden Figuren 2 bis 12 verwiesen werden.

Fig. 2 zeigt eine perspektivische Darstellung einer Detailansicht einer der Robotervorrichtungen 10 gemäß Figur 1. Da die in Figur 1 gezeigten Robotervorrichtungen 10 spiegelsymmetrisch zueinander aufgebaut sind, gelten nachfolgende Ausführungen für beide in Figur 1 gezeigten Robotervorrichtungen 10. Die Robotervorrichtung 10 umfasst neben dem distalen Bewegungsarm 14 einen proximalen Bewegungsarm 12. Der proximale Bewegungsarm 12 weist einen proximalen Armabschnitt 64 auf, der mit einer Basis 66 gekoppelt ist, sodass der proximale Bewegungsarm 12 um eine Schwenkachse S1 und/oder ein Knickgelenk, relativ zu der Basis 66 verschwenkbar ist.

Der distale Bewegungsarm 14 weist ferner einem proximalen Endabschnitt 44 auf, der über eine Gelenkeinheit 74 mit dem proximalen Bewegungsarm 12 gekoppelt ist, sodass der distale Bewegungsarm 14 um eine Drehachse D1 und/oder ein Drehgelenk relativ zu dem proximalen Bewegungsarm 12 verschwenkbar ist. Die Drehachse D1 verläuft senkrecht zu der Schwenkachse S1.

Die Robotervorrichtung 10 verfügt über einen Nebenarm 36 mit einem proximalen Armabschnitt 70 und einem distalen Armabschnitt 72. Der proximale Armabschnitt 70 des Nebenarms 36 ist über eine weitere Gelenkeinheit 76 gelenkig mit der Basis 66 verbunden. Der distale Armabschnitt 72 des Nebenarms 36 ist mit der Gelenkeinheit 74 gekoppelt. Der Nebenarm 36 und der proximale Bewegungsarm 12 verlaufen im Wesentlichen parallel zueinander. Der Nebenarm 36 und der proximale Bewegungsarm 12 sind voneinander beabstandet.

Der Nebenarm 36 kann unabhängig vom proximalen Bewegungsarm 12 auf und ab bewegt werden. Dieser Freiheitsgrad des Nebenarms 36 ist in der Fig. 2 durch einen Pfeil angedeutet. Eine derartige Auf- oder Abwärtsbewegung des Nebenarms 36 kann den distalen Bewegungsarm 14 um eine weitere Schwenkachse S2 und/oder ein weiteres Knickgelenk relativ zum proximalen Bewegungsarm 14 verschwenken. Die weitere Schwenkachse S2 verläuft parallel zu der Schwenkachse S1. Die Basis 66 der Robotervorrichtung 10 kann um eine weitere Drehachse D2 rotierbar sein. Die weitere Drehachse D2 kann parallel zu der Drehachse D1 verlaufen. Die weitere Drehachse D2 und die Drehachse D1 können auf einer gemeinsamen Achse verlaufen.

Die Robotervorrichtung 10 umfasst mehrere Interaktoren 16 mit Sensorik 78. Die Sensorik 78 ist insbesondere dazu eingerichtet, Bewegungen und/oder Bewegungsänderungen der Robotervorrichtung 10 zu detektieren und/oder aufzunehmen. Die Interaktoren 16 können derart ausgestaltet sein, dass einen physischen und/oder virtuellen Anschlag liefern, um Bewegungen der einzelnen Komponenten der Robotervorrichtung 10 um eine jeweilige Schwenk- und/oder Drehachse S1, S2, D1, D2 der jeweiligen Komponenten zuzulassen und/oder ab einem bestimmten Weg oder Betrag zu begrenzen

Ferner umfasst die Robotervorrichtung 10 eine Schnittstelle 80, die dazu eingerichtet ist, mit der Eingabevorrichtung 38 gekoppelt zu werden. Die Eingabevorrichtung 38 sind in der Fig. 2 nicht gezeigt. Gemäß Fig.2 ist die Schnittstelle 80 an einer Unterseite 82 des distalen Bewegungsarms 14 in einem Endabschnitt 40 des distalen Bewegungsarms 14 angeordnet. In anderen hier nicht gezeigten Ausführungsformen kann die Schnittstelle 80 auch an einer anderen Stelle des distalen Bewegungsarms 14, wie beispielsweise einer Oberseite 84 des distalen Bewegungsarms 14 angeordnet sein.

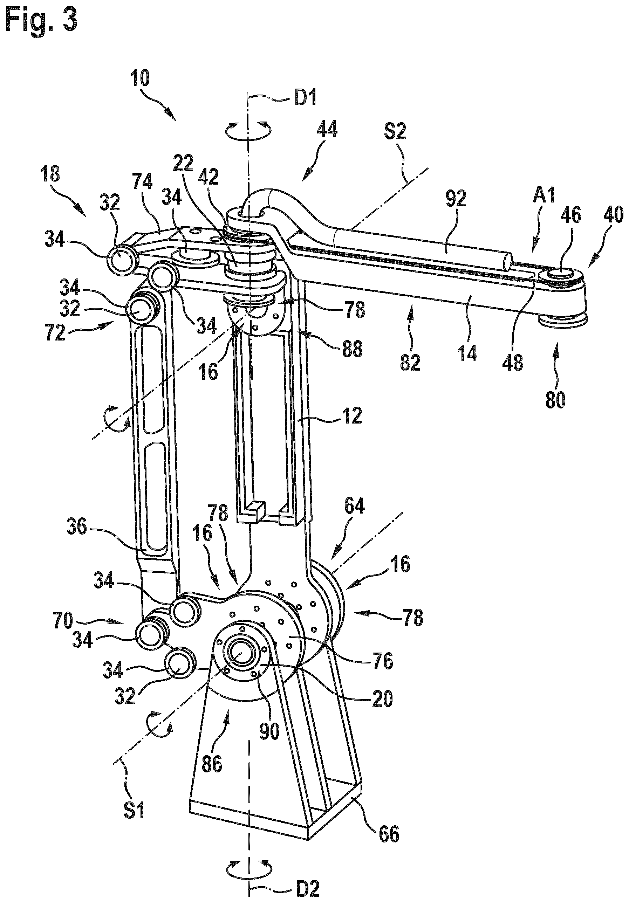

Die internen mechanischen Komponenten der Robotervorrichtung 10 sind bis auf ein erstes Kopplungselement 20 aufgrund von Verkleidungselementen 68 in der Fig. 2 nicht zu erkennen. Diesbezüglich wird auf die nachfolgende Beschreibung der Figuren 3 bis 12 verwiesen.

Fig. 3 zeigt eine perspektivische Darstellung der Robotervorrichtung 10 ohne Verkleidung 68. Zu erkennen ist eine Kopplungseinheit 18, die neben dem ersten Kopplungselement 20, ein zweites Kopplungselement 22 aufweist. Während das erste Kopplungselement 20 an einem distalen Abschnitt 86 der Basis 66 angeordnet ist, ist das zweite Kopplungselement 22 an einem distalen Armabschnitt 88 des proximalen Bewegungsarms 12, oder anders ausgedrückt zwischen dem distalen Armabschnitt 88 des proximalen Bewegungsarms 12 und dem proximalen Endabschnitt 44 des distalen Bewegungsarms 14, angeordnet. Das zweite Kopplungselement 22 ist auf der Gelenkeinheit 74 angeordnet.

Ferner umfasst die Robotervorrichtung 10 eine Zugmittelanordnung 24 mit einer Mehrzahl von Führungsmitteln 32, in Form von Führungsrollen 34, die dazu eingerichtet sind, ein Zugmittel 26 zu führen. In der hierin gezeigten Ausführungsform weist die Zugmittelanordnung 24 insgesamt sieben Führungsrollen 34 auf. Die Führungsrollen 34 sind derart angeordnet, dass das Zugmittel 26 auf unterschiedlichen Bezugsebenen geführt werden kann, wobei die Bezugsebenen zueinander senkrecht verlaufen. Das erste Kopplungselement 20 und das zweite Kopplungselement 22 sind als zylinderförmige Kabeltrommeln 90 konfiguriert und dazu eingerichtet mit dem in dieser Figur nicht gezeigten Zugmittel 26 gekoppelt zu werden, sodass eine Bewegung von einem der Kopplungselemente 20, 22 auf das jeweils andere Kopplungselement 20, 22 übertragbar ist.

Ferner zu erkennen ist ein Kabelkanal 92, der sich zumindest überwiegend entlang des distalen Bewegungsarms 14 erstreckt. Der Kabelkanal 92 kann insbesondere dazu eingerichtet sein, Kabel und/oder Leitungen, insbesondere mechanische, elektrische und/oder optische Leitungen sicher zu der Schnittstelle 80 zu führen und/oder vor äußeren Einflüssen zu schützen.

Die Robotervorrichtung 10 umfasst ferner einen Ausrichtungsmechanismus A1, der dazu eingerichtet ist, zumindest bei einer Drehbewegung des distalen Bewegungsarms 14 um die Drehachse D1 eine entsprechende Drehbewegung einer hier an dem distalen Bewegungsarm 14 anordenbaren Vorrichtung, wie beispielsweise einer Eingabevorrichtung 38 zu verhindern und/oder zu kompensieren.

Dazu umfasst der Ausrichtungsmechanismus A1 wenigstens zwei Umlenkrollen 42, 46 wobei eine erste Umlenkrolle 42 an einem proximalen Endabschnitt 44 des distalen Bewegungsarms 14 angeordnet ist, und wobei eine zweite Umlenkrolle 46 an dem distalen Endabschnitt 40 des distalen Bewegungsarms 14 angeordnet ist.

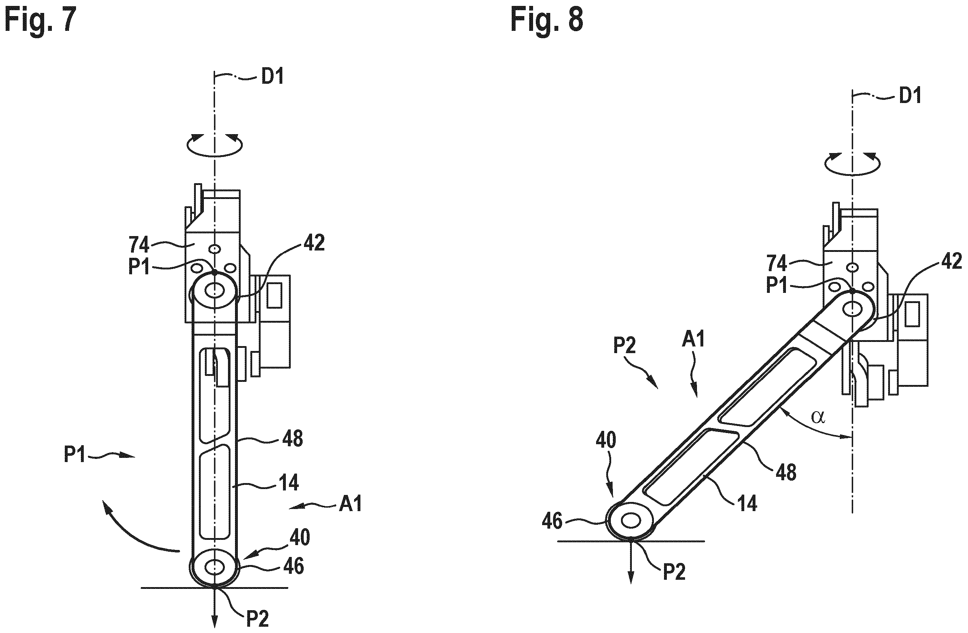

Der Ausrichtungsmechanismus A1 umfasst außerdem wenigstens ein Zugelement 48 in Form eines Seils, das zwischen den zwei Umlenkrollen 42, 46 gespannt ist, um eine Ausrichtung der Umlenkrollen 42, 46 zu synchronisieren. Die erste Umlenkrolle 42 ist relativ zur Drehachse D1 stationär. Die zweite Umlenkrolle 46 ist relativ zu der Drehachse D1 bewegbar ist. Das wenigstens eine Zugelement 48 ist an jeweils zumindest einem Punkt P1, P2 an den Umlenkrollen 42, 46 fixiert. Hinsichtlich des Funktionsprinzips des Ausrichtungsmechanismus A1 kann ferner auf die Figuren 7 bis 10 verwiesen werden.

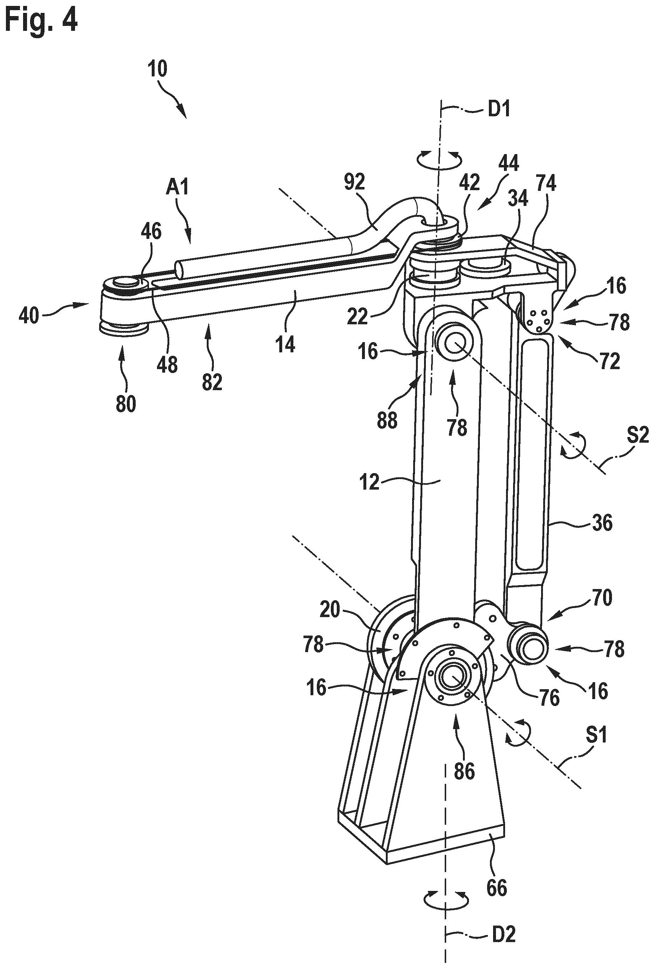

Fig. 4 zeigt eine perspektivische Darstellung der Robotervorrichtung 10 ohne Verkleidung aus einem anderen Blickwinkel.

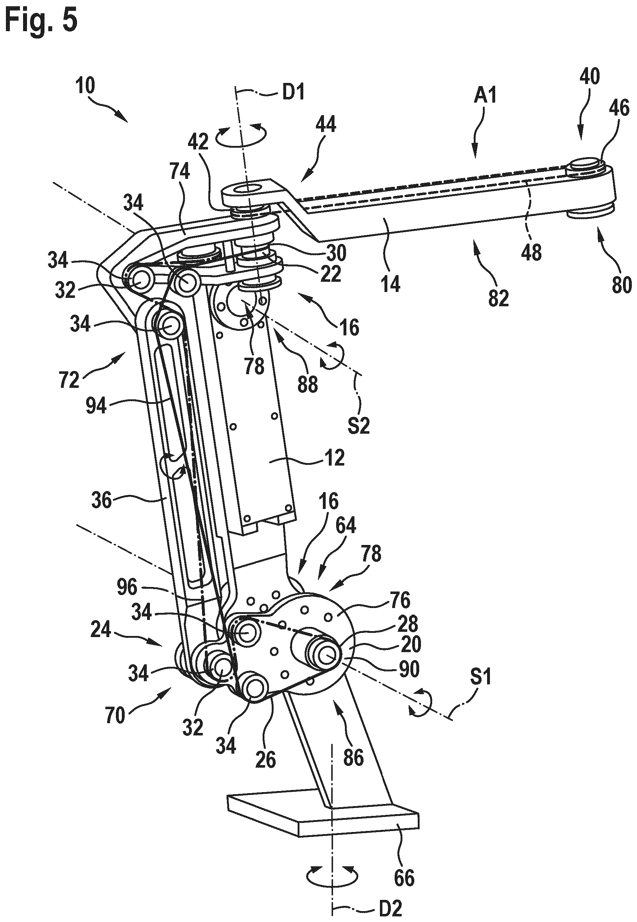

Fig. 5 zeigt eine perspektivische Darstellung der Robotervorrichtung 10 mit einem Zugmittel 26 bestehend aus einem ersten und einem zweiten Seil 94, 96. Ein erster Endabschnitt 28 eines jeweiligen Seils 94, 96 ist zumindest teilweise um das erste Kopplungselement 20 und ein zweiter Endabschnitt 30 eines jeweiligen Seils 94, 96 ist zumindest teilweise um das zweite Kopplungselement 22 gewickelt, sodass das erste Kopplungselement 20 und das zweite Kopplungselement 22 interdependent drehbar gekoppelt sind.

Die Zugmittelanordnung 24 ist zumindest teilweise an und/oder in dem Nebenarm 36 und den Gelenkeinheiten 74, 76 angeordnet.

Für eine effiziente und platzsparende Kraftübertragung zwischen den Kopplungselementen 20, 22, wird das Zugmittel 26 um die Führungsrollen 34 geführt. Jede der Gelenkeinheiten 74 und der weiteren Gelenkeinheit 76 umfasst drei Führungsrollen und bilden jeweils ein Knickgelenk. Jede Führungsrolle 34 ändert die Richtung der eines an einer jeweiligen Führungsrolle 34 umgelenkten Seils 94, 96.

Die Führungsrollen 34 sind derart angeordnet, dass das Zugmittel 26 zumindest abschnittsweise überkreuz geführt wird. Die beiden Seile 94, 96 werden kontrarotierend geführt, um eine konstante Länge des Zugmittels 26 und einen konstanten Wickelwinkel des Zugmittels 26 um das erste und zweite Kopplungselement 20, 22 in der Summe zu erreichen. Wenn der Wickelwinkel des Zugmittels 26 aufgrund einer Bewegung der Robotervorrichtung 10 um eines der Kopplungselemente 20, 22 verringert wird, erhöht sich der Wickelwinkel des Zugmittels 26 um das jeweils andere Kopplungselement 20, 22.

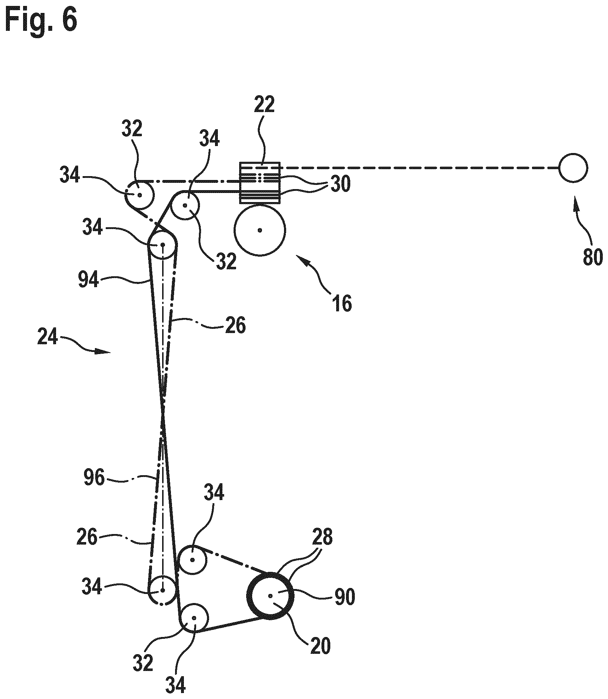

Zum besseren Verständnis ist in Fig. 6 eine schematische Darstellung der Zugmittelanordnung 24 gezeigt.

In Fig. 7 ist eine perspektivische Darstellung des distalen Bewegungsarms 14 in einer ersten Position P1 gezeigt. In Fig. 8 ist eine perspektivische Darstellung des distalen Bewegungsarms 14 in einer zweiten Position P2 gezeigt. Die zweite Position P2 unterscheidet sich von der ersten Position P1 dadurch, dass der distale Bewegungsarm 14 ausgehend von der ersten Position P1 um die Drehachse D1 verschwenkt wurde. Zu erkennen ist, dass mittels des Ausrichtungsmechanismus A1 während eines Betriebs und/oder einer Bewegung der Robotervorrichtung stets eine konstante bzw. gleiche Ausrichtung der Schnittstelle 80 und damit eines potenziell daran angeordneten Effektors 58 und/oder einer daran angeordneten Eingabevorrichtung 38 gewährleistet werden kann. Wie bereits voranstehend im Bezug auf Fig. 5 beschrieben, ist die erste Umlenkrolle 42 relativ zur Drehachse D1 stationär. Die zweite Umlenkrolle 46 ist relativ zu der Drehachse D1 bewegbar ist. Das wenigstens eine Zugelement 48 ist an jeweils zumindest einem Punkt P1, P2 an den Umlenkrollen 42, 46 fixiert. Der Gesamtwickelwinkel des Zugelements 48 an jeder der ersten und der zweiten Umlenkrolle 42, 46 beträgt 180°. Sobald der distale Bewegungsarm 14 um die Drehachse D1 geschwenkt wird, verschiebt sich der Abschnitt, in dem das Zugelement 48 um eine der Umlenkrollen 42, 46 gewickelt ist und/oder diese kontaktiert, in eine zu der Schwenkrichtung entgegengesetzten Richtung.

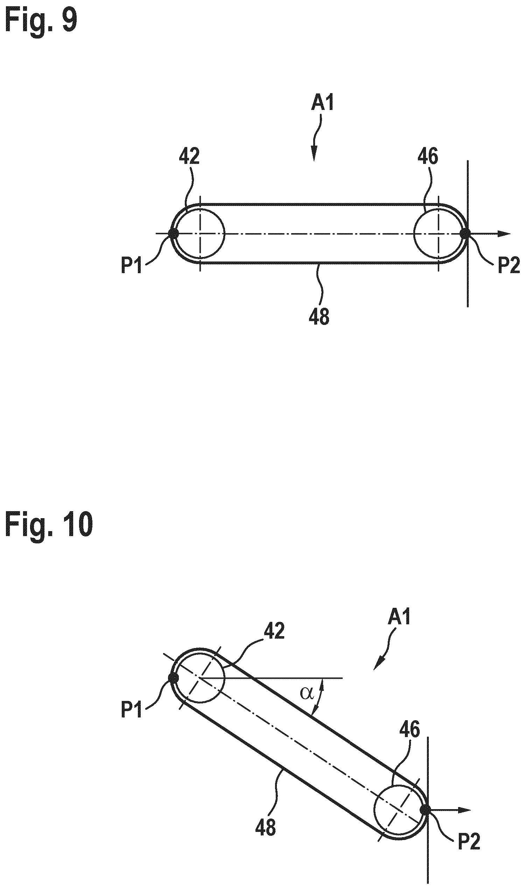

Zur besseren Veranschaulichung ist das Funktionsprinzip des Ausrichtungsmechanismus A1 in den Figuren 9 und 10 nochmals schematisch dargestellt. Fig. 9 zeigt eine schematische Darstellung des Ausrichtungsmechanismus A1 in der ersten Position P1. Fig. 10 zeigt eine schematische Darstellung des Ausrichtungsmechanismus in der zweiten Position P2, in welcher der distale Bewegungsarm 14 um einen Winkelbetrag α verschwenkt wurde.

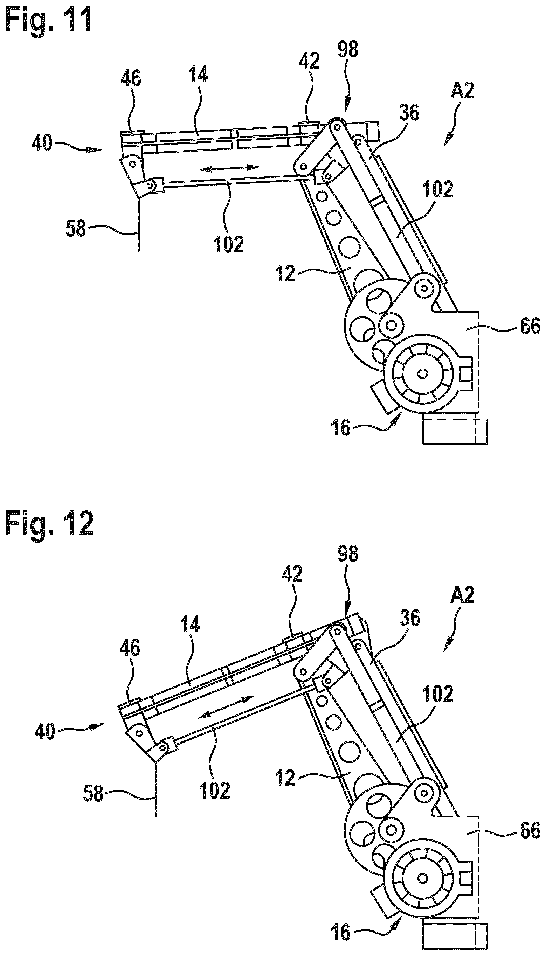

In den Figuren 11 und 12 ist jeweils eine Seitenansicht einer Robotervorrichtung 10 gemäß einer weiteren Ausführungsform gezeigt. Die in den Figuren 11 und 12 gezeigte Ausführungsform ist insbesondere für einen Einsatz in einer Slave-Einheit eingerichtet. Gemäß dieser Ausführungsform umfasst die Robotervorrichtung 10 alternativ zu dem vorangehend beschriebenen Ausrichtungsmechanismus A1 einen Ausrichtungsmechanismus A2. Dieser Ausrichtungsmechanismus A2 verwendet eine spezielle parallele Kinematik, um die Ausrichtung eines an die Robotervorrichtung 10 gekoppelten Effektors auszurichten und/oder um die Ausrichtung des Effektors 10 gegenüber einem Benutzer konstant zu halten, unabhängig von der Positionierung der Bewegungsarme 12, 14 der Robotervorrichtung 10. Dazu umfasst der Ausrichtungsmechanismus A2 eine Zug- und/oder Druckstangenanordnung 98. Die Zug- und oder Druckstangenanordnung 98 kann mehrere Zug- und/oder Druckstangen 102 umfassen, die bewegungsübertragbar miteinander sowie dem Effektor 58 gekoppelt sind. Die Zug- und/oder Druckstangenanordnung 98 kann sich ausgehend von einer Basis 66 der Robotervorrichtung 10 und entlang und/oder parallel eines proximalen und/oder eines distalen Bewegungsarms 14 erstrecken.

Das Funktionsprinzip des weiteren Ausrichtungsmechanismus A2 ist in den Figuren 11 und 12 gezeigt. Mittels linearer Bewegungen der Zug- und/oder Druckstangen 102 kann der Effektor 58 unabhängig von den Bewegungsarmen 12, 14 ausgerichtet werden.

### Bezugszeichenliste

- 10: Robotervorrichtung
- 12: proximaler Bewegungsarm
- 14: distaler Bewegungsarm
- 16: Interaktor
- 18: Kopplungseinheit
- 20: erstes Kopplungselement
- 22: zweites Kopplungselement
- 24: Zugmittelanordnung
- 26: Zugmittel
- 28: erster Endabschnitt des Zugmittels
- 30: zweiter Endabschnitt des Zugmittels
- 32: Führungsmittel
- 34: Führungsrolle
- 36: Nebenarm
- 38: Eingabevorrichtung
- 40: distaler Endabschnitt des distalen Bewegungsarms
- 42: erste Umlenkrolle
- 44: proximaler Endabschnitt des distalen Bewegungsarms
- 46: zweite Umlenkungsrolle
- 48: Zugelement
- 52: Master-Einheit
- 58: Effektor
- 60: Anzeigeeinheit
- 62: Bild
- 64: proximaler Armabschnitt des proximalen Bewegungsarms
- 66: Basis
- 68: Verkleidungselement
- 70: proximaler Armabschnitt des Nebenarms
- 72: distaler Armabschnitt des Nebenarms
- 74: Gelenkeinheit
- 76: weitere Gelenkeinheit
- 78: Sensorik
- 80: Schnittstelle
- 82: Unterseite des distalen Bewegungsarms
- 84: Oberseite des distalen Bewegungsarms
- 86: distaler Abschnitt der Basis
- 88: distaler Armabschnitt des proximalen Bewegungsarms
- 90: Kabeltrommel
- 92: Kabelkanal
- 94: erstes Seil
- 96: zweites Seil
- 98: Zug- und/oder Druckstangenanordnung
- 100: medizinisches Robotersystem
- 102: Zug- und/oder Druckstange
- D1: Drehachse
- D2: weitere Drehachse
- S1: Schwenkachse
- S2: weitere Schwenkachse
- A1: Ausrichtungsmechanismus
- A2: weiterer Ausrichtungsmechanismus
- P1: erster Fixierungspunkt
- P2: zweiter Fixierungspunkt
- α: Winkelbetrag

## Patentansprüche

1. Robotervorrichtung (10) umfassend:
- einen proximalen Bewegungsarm (12), der um wenigstens eine Schwenkachse (S1) verschwenkbar ist;
- einen distalen Bewegungsarm (14), der gelenkig mit dem proximalen Bewegungsarm (12) gekoppelt ist,
wobei der distale Bewegungsarm (14) um wenigstens eine Drehachse (D1) relativ zum proximalen Bewegungsarm (12) drehbar ist, wobei die Drehachse (D1) winklig zu der Schwenkachse (S1) verläuft.

2. Robotervorrichtung (10) nach Anspruch 1, weiter umfassend
- einen Interaktor (16) und
- eine Kopplungseinheit (18), welche ein mit dem Interaktor (16) verbundenes erstes Kopplungselement (20), ein mit dem distalen Bewegungsarm (14) verbundenes zweites Kopplungselement (22) und eine Zugmittelanordnung (24) mit wenigstens einem Zugmittel (26) aufweist, welches mit dem ersten Kopplungselement (20) und dem zweiten Kopplungselement (22) gekoppelt ist, sodass eine Bewegung von einem der Kopplungselemente (20, 22) auf das andere der Kopplungselemente (20, 22) übertragbar ist.

3. Robotervorrichtung (10) nach Anspruch 2,
wobei ein erster Endabschnitt (28) des Zugmittels (26) zumindest teilweise um das erste Kopplungselement (20) und ein zweiter Endabschnitt (30) des Zugmittels (26) zumindest teilweise um das zweite Kopplungselement (22) gewickelt ist, sodass das erste Kopplungselement (20) und das zweite Kopplungselement (22) interdependent drehbar gekoppelt sind.

4. Robotervorrichtung (10) nach Anspruch 2 oder 3,
wobei die Zugmittelanordnung (24) eine Mehrzahl von Führungsmitteln (32), vorzugsweise Führungsrollen (34), umfasst, die dazu eingerichtet sind, das Zugmittel (26) zu führen.

5. Robotervorrichtung (10) nach Anspruch 4,
wobei die Führungsmittel (32) derart angeordnet sind, dass sich das Zugmittel (26) zumindest abschnittsweise überkreuzt.

6. Robotervorrichtung (10) nach einem der Ansprüche 2 bis 5,
wobei das erste Kopplungselement (20) und das zweite Kopplungselement (22) jeweils um Rotationsachsen drehbar sind, welche winklig zueinander angeordnet sind.

7. Robotervorrichtung (10) nach einem der vorherigen Ansprüche, wobei der distale Bewegungsarm (14) relativ zum proximalen Bewegungsarm (12) um eine weitere Schwenkachse (S2) schwenkbar ist.

8. Robotervorrichtung (10) nach Anspruch 7,
wobei die weitere Schwenkachse (S2) parallel zur Schwenkachse (S1) verläuft.

9. Robotervorrichtung (10) nach Anspruch 7 oder 8,
wobei die weitere Schwenkachse (2) senkrecht zur Drehachse (D1) verläuft.

10. Robotervorrichtung (10) nach einem der Ansprüche 7 bis 9, weiter umfassend:
- einen Nebenarm (36), welcher zumindest abschnittsweise entlang des proximalen Bewegungsarms (12) verläuft und von diesem beabstandet ist und welcher bei einer Schwenkbewegung des distalen Bewegungsarms (14) um die weitere Schwenkachse (2) relativ zum proximalen Bewegungsarm (12) bewegbar ist.

11. Robotervorrichtung (10) zumindest nach den Ansprüchen 2 und 10, wobei die Zugmittelanordnung (24) zumindest teilweise an und/oder in dem Nebenarm (36) angeordnet ist.

12. Robotervorrichtung (10) nach einem der vorherigen Ansprüche, ferner umfassend:
- eine Eingabevorrichtung (38), die dazu eingerichtet ist, eine Bewegung und/oder Befehle eines Benutzers aufzunehmen,
wobei die Eingabevorrichtung (38) an einem distalen Endabschnitt (40) des distalen Bewegungsarms (14) angeordnet ist.

13. Robotervorrichtung (10) nach Anspruch 12, ferner umfassend:
- einen Ausrichtungsmechanismus (A1), der dazu eingerichtet ist, zumindest bei einer Drehbewegung des distalen Bewegungsarms (14) um die Drehachse (D1) eine entsprechende Drehbewegung der Eingabevorrichtung (38) zu verhindern und/oder zu kompensieren.

14. Robotervorrichtung (10) nach Anspruch 13,
wobei der Ausrichtungsmechanismus (A1) umfasst:
- wenigstens zwei Umlenkrollen (42), wobei eine erste Umlenkrolle (42) an einem proximalen Endabschnitt (44) des distalen Bewegungsarms (14) angeordnet ist, und wobei eine zweite Umlenkrolle (46) an dem distalen Endabschnitt (40) des distalen Bewegungsarms (14) angeordnet ist,
- wenigstens ein Zugelement (48), das zwischen und/oder um die wenigstens zwei Umlenkrollen (42, 46) gespannt ist, um eine Ausrichtung der Umlenkrollen (42, 46) zu synchronisieren.

15. Robotervorrichtung (10) nach einem der Ansprüche 12 bis 14, wobei die Eingabevorrichtung (38) drehfest an der zweiten Umlenkrolle (46) angeordnet ist.

16. Robotervorrichtung (10) nach Anspruch 14 oder 15,
wobei die erste Umlenkrolle (42) relativ zur Drehachse (D1) stationär und die zweite Umlenkrolle (46) relativ zu der Drehachse (D1) bewegbar ist.

17. Robotervorrichtung (10) nach einem der Ansprüche 14 bis 16, wobei das wenigstens eine Zugelement (48) an jeweils zumindest einem Punkt (P1, P2) an den Umlenkrollen (42, 46) fixiert ist.

18. Medizinisches Robotersystem (100) umfassend:
- eine Robotervorrichtung (10) nach einem der vorherigen Ansprüche.
